Europäisches Patentamt

European Patent Office

Office européen des brevets

(19)

(11) Numéro de publication : **0 449 719 A1**

## (12) DEMANDE DE BREVET EUROPEEN

(21) Numéro de dépôt : **91400805.7**

(51) Int. Cl.$^5$ : **G02B 6/12, G02F 1/29**

(22) Date de dépôt : **26.03.91**

(30) Priorité : **27.03.90 FR 9003878**

(43) Date de publication de la demande :
**02.10.91 Bulletin 91/40**

(84) Etats contractants désignés :
**DE GB**

(71) Demandeur : **THOMSON-CSF**
**51, Esplanade du Général de Gaulle**
**F-92800 Puteaux (FR)**

(72) Inventeur : **de Monts, Humbert,**
**THOMSON-CSF**
**SCPI - CEDEX 67**
**F-92045 Paris la Défense (FR)**

(74) Mandataire : **Guérin, Michel et al**
**THOMSON-CSF SCPI**
**F-92045 PARIS LA DEFENSE CEDEX 67 (FR)**

(54) **Structure guidante intégrée en trois dimensions, et son procédé de réalisation.**

(57) L'invention concerne, un coupleur optique dans lequel deux guides (G1,G2) réalisés dans l'épaisseur d'un substrat sont situés dans deux plans distincts (P1,P2). Ils ont des configurations sinueuses de façon à présenter au moins une zone de couplage (C) perpendiculairement aux plans (P1,P2) des guides.
Application : commutation optique.

FIG.1

EP 0 449 719 A1

L'invention concerne une structure guidante intégrée en trois dimensions et plus particulièrement une structure réalisée en matériau semiconducteur ainsi que son procédé de réalisation.

La réalisation de guides prévoyant un couplage entre guides demande une précision qui est difficile à obtenir dans les techniques connues en raison des limitations des techniques de microlithographie.

L'invention permet de réaliser des coupleurs optiques dont on peut régler avec précision la distance des guides dans les zones de couplage.

De plus, les semiconducteurs présentent actuellement un grand potentiel comme matériaux de base pour la réalisation de composants optoélectroniques. Les techniques de croissance (MOCVD) ou (MBE) permettent le contrôle des épaisseurs des couches à quelques Angströms près et la croissance de différents types de semiconducteurs sur le même substrat. Les interfaces entre deux couches de semiconducteurs différents peuvent être abruptes et de très bonne qualité cristallographique induisant de très faibles pertes optiques. Actuellement les pertes à la propagation sont inférieures à 0,2dB/cm (voir le document : "Very low loss waveguides and Efficient Modulators in InGaAsP/InP" de Y. Bourbin et al dans Proceedings of the IGWO conference in Boston USA, February 1989, pages 110-112).

D'autre part les techniques de microlithographie et les procédés d'attaque chimique sélective peuvent, à l'heure actuelle, permettre la réalisation de motifs de la taille du micron. L'attaque chimique sélective est contrôlée grâce à une couche d'arrêt et il est courant de réaliser des rubans d'épaisseur de quelques dizaines d'amgstoms et de largeur de l'ordre d'un micron sur une longueur de plusieurs centimètres.

Ainsi, l'association de ces deux techniques permet actuellement la réalisation de la structure décrite dans la présente invention.

L'état de l'art des composants optoélectroniques permet de réaliser les fonctions que peut remplir la structure décrite dans l'invention, mais la compacité, la performance et la multifonctionnalité de la structure et son procédé de réalisation très simple font l'intérêt de l'invention.

L'invention donne une solution au problème de l'intégration en trois dimensions car il y a l'utilisation de la dimension verticale dans l'invention. L'idée est d'utiliser le couplage de la lumière verticalement dans une structure bimodale et d'utiliser les procédés de lithographie pour les deux dimensions du plan.

La multifonctionnalité vient des propriétés des coupleurs directionnels avec comme particularité dans le cas de cette structure, le fait d'avoir des longueurs de couplage très courtes. Ceci est un avantage important pour le multiplexage ou le démultiplexage en longueur d'onde. Par contre pour la modulation de lumière les effets peuvent être renforcés par la présence dans la structure de matériau très actif comme les MPQ (Multi Puits Quantiques).

La structure de l'invention présente donc les avantages suivants :
- l'intégration en trois dimensions,
- la multifonctionnalité,
- la performance,
- la facilité de réalisation,
- la simplicité.

L'invention concerne donc une structure guidante intégrée en trois dimensions, caractérisée en ce qu'elle comporte au moins deux guides d'ondes disposés respectivement dans un premier et un deuxième plans et possédant au moins une zone de couplage dans laquelle au moins une portion d'un premier guide est proche d'une portion d'un deuxième guide.

Plus particulièrement, l'invention concerne une structure guidante, caractérisée en ce qu'elle comporte :
- une première couche tampon d'un premier indice ;
- un premier ruban d'un deuxième indice supérieur au premier indice, située sur la première couche ;
- une couche guidante d'un troisième indice inférieur au deuxième indice recouvrant le premier ruban et la première couche tampon ;
- une deuxième couche tampon d'un quatrième indice recouvrant la couche guidante ;
- un deuxième ruban d'un cinquième indice supérieur au quatrième indice situé sur la deuxième couche tampon ;
- une troisième couche tampon d'un sixième indice inférieur au cinquième indice recouvrant le deuxième ruban et la deuxième couche tampon.

De plus, l'invention concerne un procédé de réalisation d'une structure guidante selon la revendication 4, caractérisé en ce qu'il comporte les étapes suivantes ;
- une première étape de réalisation sur un substrat de la première couche tampon ;
- une deuxième étape de réalisation d'une couche d'un matériau d'indice supérieur à celui de la première couche tampon suivie d'une gravure du premier ruban ;
- une troisième étape de réalisation sur le premier ruban et sur la première couche tampon de la couche guidante ;
- une quatrième étape de réalisation sur la couche guidante d'une deuxième couche tampon ;
- une cinquième étape de réalisation d'une couche d'un matériau d'indice supérieur à celui de la deuxième couche tampon suivie d'une gravure du deuxième ruban ;
- une sixième étape de réalisation d'une troisième couche tampon sur le deuxième ruban et la deuxième couche tampon.

Les différents objets et caractéristiques de l'invention apparaîtront plus clairement dans la description qui va suivre et des figures annexées qui représentent :

- la figure 1, une forme de réalisation générale du dispositif de l'invention ;
- la figure 2, une forme de réalisation détaillée à l'aide de matériaux semiconducteurs du dispositif de l'invention ;
- les figures 3 à 5, des vues détaillées du dispositif de la figure 2 ;
- les figures 6 et 7, des exemples de réalisation des différentes couches selon l'invention ;
- la figure 8, une variante de réalisation en vue de dessus du dispositif de l'invention.

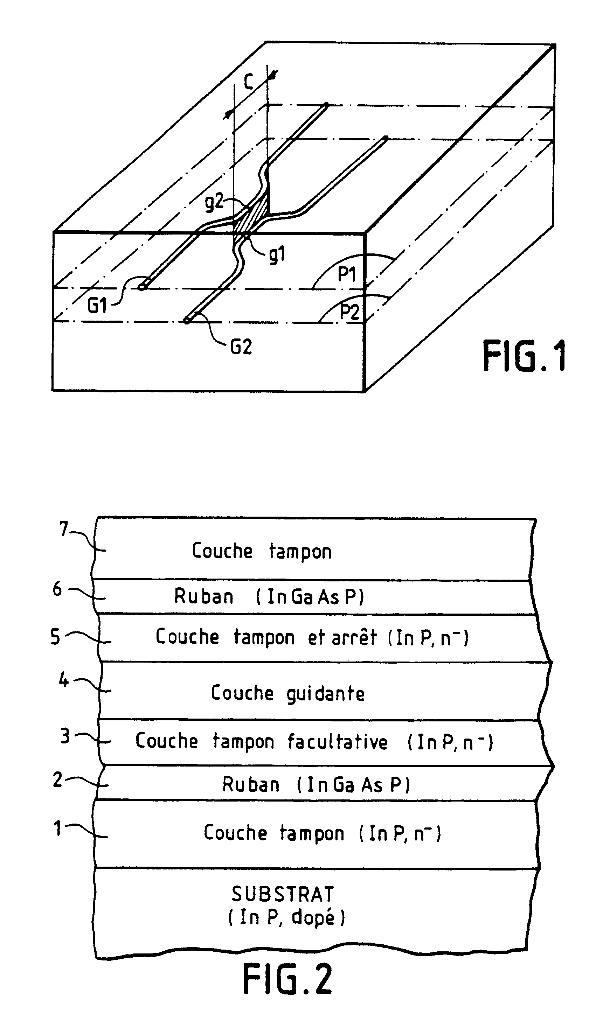

En se reportant à la figure 1, on va tout d'abord décrire une forme de réalisation générale de la structure de l'invention. Cette structure comporte un premier guide G1 situé dans un plan P1 d'un substrat. Un deuxième guide G2 est situé dans un plan P2 parallèle au plan P1. Les deux guides comportent des portions g1 et g2 qui sont proches l'une de l'autre et qui constituent une zone de couplage C. De préférence, ces portions g1 et g2 sont situées dans un plan perpendiculaire aux plans P1 et P2. Dans ces conditions, les portions g1 et g2 sont séparées par la distance existant entre les plans P1 et P2. La distance entre les plans P1 et P2, qui peut être déterminée aisément avec précision, caractérise le couplage entre les portions g1 et g2 des guides G1 et G2.

En se reportant à la figure 2, on va maintenant décrire un dispositif selon l'invention réalisé en matériau semiconducteur. Le matériau semiconducteur sera, à titre d'exemple, à base d'indium et de phosphore.

Le dispositif comporte sur un substrat S en InP dopé, une couche tampon 1 en InP dopé $n^-$.

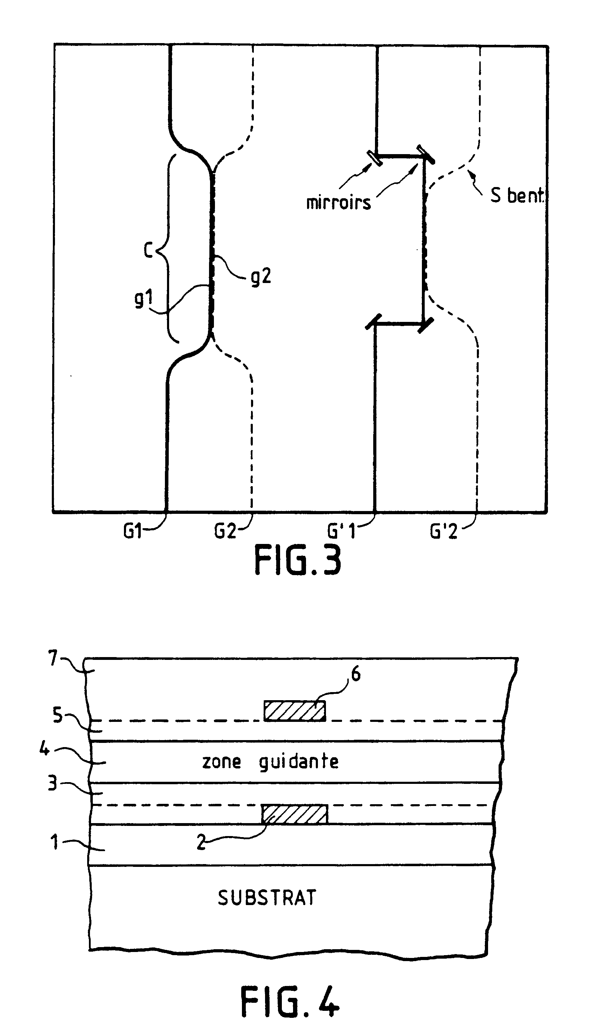

Sur cette couche tampon est réalisé un ruban 2, en InGaAsP par exemple, d'indice de réfraction supérieur au matériau de la couche tampon 1. La forme de ce ruban 2 a une forme telle représentée en figure 3 représentant une vue du dessus du dispositif.

L'ensemble couche tampon 1 et ruban 2 est recouvert par une couche tampon (3) en InP dopé $n^-$. cette couche tampon n'est cependant pas nécessaire dans tous les cas.

Une couche guidante 4 en InGaAsP recouvre la couche tampon (3).

Une couche tampon 5 en InP dopé $n^-$ recouvre la couche guidante 4.

Sur cette couche tampon 5 est réalisé un deuxième ruban 6 en InP d'indice supérieur au matériau de la couche 5.

La forme du ruban 6 a une forme telle que représentée en figure 3.

Enfin, la couche tampon 5 et le ruban 6 sont recouverts par une couche tampon 7 en InP.

Chaque guide présente une structure enterrée telle que décrite dans la demande de brevet n° 86 04523. Les deux guides sont donc enterrés à des niveaux différents et sont situés dans des plans différents de la structure.

Les matériaux utilisés pour les différentes couches tampon seront de préférence identiques. De même, les deux rubans seront de natures identiques.

Les formes non linéaires des rubans représentées en figure 3 permettent de réaliser des couplages entre les guides tels que dans la zone de couplage C.

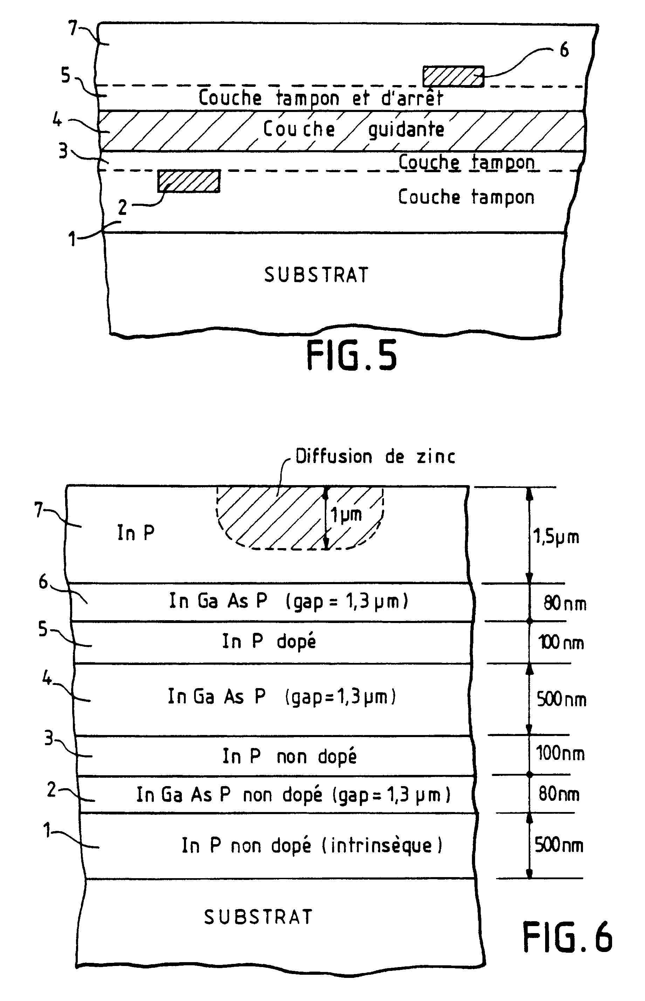

Un tel coupleur permet un raccordement aisé des entrées/sorties des guides. En effet, sur la figure 4 représentant une coupe perpendiculaire aux guides, les accès aux guides sont parfaitement distincts. Par contre, la figure 5 représente une coupe perpendiculaire dans la zone de couplage C.

La structure décrite précédemment peut fonctionner sous deux modes : passif ou actif c'est-à-dire avec la possibilité d'appliquer un champ électrique ou injecter un courant.

En tant que composant passif, cette structure permet la séparation des longueurs d'onde avec un pouvoir séparateur très important grâce à la très courte longueur de couplage de l'ordre de plusieurs dizaines de micromètres. Réciproquement, cette structure permet grâce aux guides d'accès de multiplexer les longueurs d'onde. Aussi la structure permet de connecter deux points d'un volume. En effet les trois directions de déflection du faisceau lumineux sont rendues possible d'une part par la lithographie pour les deux directions horizontales et d'autre part verticalement par l'échange d'énergie entre les deux guides superposés.

Ce composant peut être soumis à un champ électrique, en prévoyant des électrodes non représentées au-dessus des guides, et avoir ainsi un fonctionnement dépendant du champ appliqué.

On peut utiliser le composant en tant que commutateur de lumière. Le champ électrique va pour cela perturber l'échange d'énergie et donc la longueur de couplage entre les deux guides. Suivant l'application d'un champ électrique l'énergie lumineuse commutera d'un guide de sortie à l'autre. On peut modifier les indices des couches par application d'un champ électrique ou par injection de charges.

Le champ électrique permet aussi de régler le fonctionnement de la structure en multiplexeur ou démultiplexeur à la longueur d'onde d'utilisation.

Pour augmenter les effets de champ électrique sur la structure, la couche guidante principale (4) peut être une structure à Multi Puits Quantiques (MPQ). Cette couche MPQ donne une liberté supplémentaire au choix de l'indice optique et des effets physiques différents et importants en mode actif.

Dans la structure, il se crée sous l'application du champ électrique, une zone dépeuplée en porteur libre non uniforme dont les effets sont très importants et peuvent être utilisés pour le fonctionnement du composant.

La figure 6 représente un exemple de réalisation détaillé du dispositif de l'invention.

Les différents éléments mentionnés précédemment ont les caractéristiques (matériaux et épaisseurs) suivantes :

– substrat : InP dopé $n^+$
– couche tampon 1 : InP non dopé (intrinsèque) ; épaisseur 500 nm
– ruban 2 : InGaAsP non dopé ayant une largeur de bande d'énergie de 953 meV ; épaisseur 80 nm
– couche tampon 3 : InP non dopé ; épaisseur 100 nm
– couche guidante principale 4 : InGaAsP non dopé ayant une largeur de bande d'énergie de 953 meV ; épaisseur 500 nm
– couche tampon 5 : InP non dopé, épaisseur 100 nm
– ruban 6 : InGaAsP de largeur de bande d'énergie de 953 meV ; épaisseur 80 nm
– couche tampon 7 : InP dopé p ; épaisseur 1500 nm.

La couche tampon 7 est dopée de type p, par diffusion de Zinc par exemple, sur environ 1000 nm de profondeur environ, pour permettre d'établir un contact p.

Une telle structure présente des couches 3 et 5 quasiment identiques entre elles et des rubans 2 et 6 également quasiment identiques entre eux. Cette structure est donc symétrique par rapport à la couche guidante principale 4.

Un exemple de composition des rubans 2 et 6 est le suivant :

$In_{0,72} Ga_{0,28} As_{0,6} P_{0,4}$

Selon une variante de réalisation de l'invention, la couche guidante principale 4 peut constituer un multipuits quantique. Par exemple, ce multipuits quantique peut être une alternance de couche de InGaAsP et d'InP.

Plus précisément, par exemple, il peut être constitué de :

– 30 couches de InGaAsP de largeur de bande d'énergie de 1,55 µm, de formule

$In_{0,58} Ga_{0,42} As_{0,9} P_{0,1}$, d'épaisseur 8 nm chacune.
– 30 couches d'InP d'épaisseur 10 nm.

Une telle couche guidante 4 a donc pour épaisseur de 540 nm.

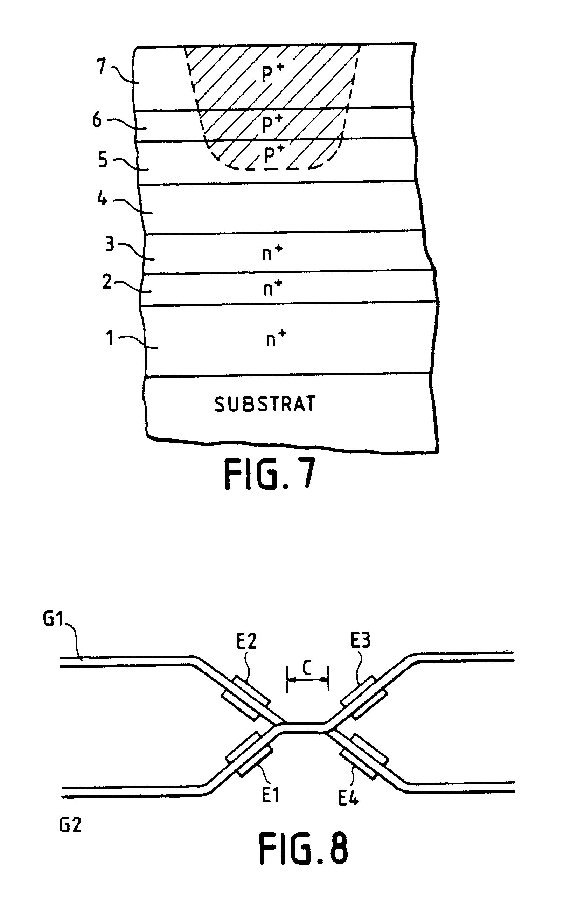

Pour augmenter le champ électrique dans la couche guidante principale, on peut doper $n^+$ les couches 1,2 et 3 et faire une diffusion plus profonde $p^+$ à travers les couches 7,6 et 5 comme cela est représenté en figure 7.

La zone intrinsèque 4 et les couches 3 et 5 vont se dépeupler sous une tension inverse de la diode p/n et créer des variations d'indice.

Selon l'exemple de réalisation de la figure 8, les deux guides G1 et G2 peuvent être réalisés de façon à se croiser. Egalement les électrodes (E1,E2,E3,E4) d'applications de champs électriques peuvent être situées sur les guides de part et d'autre du croisement à proximité du croisement.

On va maintenant décrire un procédé de réalisation du dispositif de l'invention.

A partir d'un substrat semiconducteur (par exemple InP), on dépose par les techniques de dépôt (MOCVD) ou (MBE), une couche tampon 1, puis une deuxième couche d'un matériau différent et d'indice supérieure à la couche 1, cette couche pourra ainsi être attaquée par une solution chimique et sera le ruban 2 permettant le confinement latéral. Ce ruban aura la forme que lui donnera le masque avec une zone d'interaction et une partie en forme de S ou muni de miroirs pour permettre l'accès ou la sortie du signal.

Après cette étape, l'échantillon est replacé dans un bâti de croissance pour une reprise d'épitaxie ce bâti peut être différent du premier bâti. Ensuite c'est la couche 3 qui est déposée comme couche tampon. Cette couche n'est pas indispensable mais elle permet de rendre la structure symétrique si on le désire. Ensuite la couche guidante 4 d'indice optique supérieure à la couche tampon est déposée. Cette couche peut être du même matériau que la couche 2 ou bien d'un autre type de matériau semiconducteur ou bien composée d'un empilement de couche de deux types de semiconducteur formant ainsi une structure : Multi Puits Quantiques. Puis on dépose une couche 5, couche tampon et couche d'arrêt et enfin une autre couche 6 d'indice supérieur aux couches tampon. Cette couche peut être formée à partir d'un matériau différent de 2 suivant que l'on veut une structure symétrique ou non.

L'étape suivante est une attaque chimique sélective de la couche 6 localisée définissant un ruban ayant la forme planaire décrite par le masque. Le traitement de cette couche 6 est similaire à celui de la couche 2.

L'échantillon est replacé dans un bâti de croissance pour un dépôt d'une couche tampon 7 afin d'enterrer la structure pour diminuer les pertes à la propagation et faciliter les étapes technologiques qui sont nécessaires aux contacts électriques.

**Revendications**

1. Structure guidante intégrée en trois dimensions, caractérisée en ce qu'elle comporte au moins deux guides d'onde (G1, G2) disposés respectivement dans un premier et un deuxième plans (P1, P2) et possédant au moins une zone de couplage (C) dans laquelle au moins une portion (g1) d'un premier guide (G1) est proche d'une portion (g2) d'un deuxième guide (G2).

2. Structure guidante selon la revendication 1, caractérisée en ce que les premier et deuxième plans sont parallèles entre eux et que les portions (g1 et g2) des deux guides (G1 et G2) sont situées dans un troisième plan perpendiculaire aux premier et deuxième plans (P1, P2).

3. Structure guidante selon la revendication 1, caractérisée en ce que, en dehors des portions (g1, g2) de la zone de couplage (C), les guides sont contenus dans deux plans sensiblement équidistants au troisième plan et perpendiculaires aux premier et deuxième plans (P1, P2).

4. Structure guidante selon la revendication 1, caractérisée en ce qu'elle comporte :
    – une première couche tampon (1) d'un premier indice (n1) ;
    – un premier ruban (2) d'un deuxième indice (n2) supérieur un premier indice, située sur la première couche (1) ;
    – une couche guidante (4) d'un troisième indice (n4) inférieur au deuxième indice (n2) recouvrant le premier ruban (2) et la première couche tampon (1) ;
    – une deuxième couche tampon (5) d'un quatrième indice (n5) recouvrant la couche guidante (4) ;
    – un deuxième ruban (6) d'un cinquième indice (n6) supérieur au quatrième indice situé sur la deuxième couche tampon (5) ;
    – une troisième couche tampon (7) d'un sixième indice (n7) inférieur au cinquième indice (n6) recouvrant le deuxième ruban (6) et la deuxième couche tampon (5).

5. Structure guidante selon la revendication 4, caractérisée en ce qu'elle comporte une quatrième couche tampon (3) située entre le premier ruban (2) et la couche guidante (4).

6. Structure guidante selon la revendication 4, caractérisée en ce que les première, deuxième, troisième et quatrième couches tampon (1,5,7,3) sont en matériaux similaires.

7. Structure guidante selon la revendication 6, caractérisée en ce que les rubans (2,6) sont en matériaux similaires d'indices supérieurs aux matériaux des couches tampon (1,3,5,7).

8. Structure guidante selon la revendication 5, caractérisée en ce que les deuxième et quatrième couches (3,5) sont quasiments identiques en compositions, dopages et épaisseurs et que les rubans (2,6) sont également quasiment identiques en compositions, dopages et dimensions de façon à ce que la structure présente une symétrie par rapport à la couche guidante (4).

9. Structure guidante selon la revendication 4, caractérisée en ce que la couche guidante (4) est réalisée sous la forme d'une alternance de couches constituant un multipuits quantique.

10. Structure guidante selon la revendication 4, caractérisée en ce que les premier et deuxième rubans forment une zone de couplage (C) dans laquelle les rubans présentent des portions situées dans un plan perpendiculaire aux plans des couches (1, 4, 6, 7).

11. Procédé de réalisation d'une structure guidante selon la revendication 4, caractérisé en ce qu'il comporte les étapes suivantes :
    – une première étape de réalisation sur un substrat de la première couche tampon (1),
    – une deuxième étape de réalisation d'une couche d'un matériau d'indice supérieur à celui de la première couche tampon (1) suivie d'une gravure du premier ruban (2) ;
    – une troisième étape de réalisation sur le premier ruban (2) et sur la première couche tampon (1) de la couche guidante (4) ;
    – une quatrième étape de réalisation sur la couche guidante (4) d'une deuxième couche tampon (5) ;
    – une cinquième étape de réalisation d'une couche d'un matériau d'indice supérieur à celui de la deuxième couche tampon (5) suivie d'une gravure du deuxième ruban (6) ;
    – une sixième étape de réalisation d'une troisième couche tampon (7) sur le deuxième ruban (6) et la deuxième couche tampon (5) ;

12. Procédé selon la revendication 11, caractérisé en qu'il comporte entre la deuxième étape et la troisième étape, une étape supplémentaire de réalisation d'une quatrième couche tampon (3) en matériau d'indice inférieur à celui de la couche guidante (4).

13. Procédé selon la revendication 12, caractérisé en ce que les étapes de réalisation de couches utilisent des techniques de dépôt par MOCVD (Epitaxie d'organométalliques en phase vapeur) MBE (epitaxie par faisceau moléculaire).

14. Procédé selon la revendication 12, caractérisé en ce que les opérations de gravures des premier et deuxième rubans (2, 6) se font par gravure chimique sélective.

FIG.1

7 — Couche tampon

6 — Ruban ( In Ga As P )

5 — Couche tampon et arrêt (In P, n⁻)

4 — Couche guidante

3 — Couche tampon facultative ( In P, n⁻)

2 — Ruban ( In Ga As P )

1 — Couche tampon ( In P, n⁻)

SUBSTRAT
( In P, dopé )

FIG.2

FIG.3

FIG.4

7

7
6

5
Couche tampon et d'arrêt
4
Couche / guidante
3
Couche tampon
Couche tampon
2
1

SUBSTRAT

## FIG.5

Diffusion de zinc

7
In P
1µm
1,5µm

6
In Ga As P (gap = 1,3 µm)
80 nm
5
In P dopé
100 nm
4
In Ga As P (gap = 1,3 µm)
500 nm
3
In P non dopé
100 nm
2
In Ga As P non dopé (gap = 1,3 µm)
80 nm
1
In P non dopé (intrinsèque)
500 nm

SUBSTRAT

## FIG.6

FIG. 7

FIG.8

Office européen
des brevets

# RAPPORT DE RECHERCHE EUROPEENNE

Numero de la demande

EP 91 40 0805

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | DE-A-3 815 293 (LICENTIA PATENT-VERWALTUNGS-GmbH) * Colonne 1, ligne 51 - colonne 5, ligne 28; figures 1-5 * | 1,2,4, 11,12, 13 | G 02 B  6/12 G 02 F  1/29 |
| X | JOURNAL OF APPLIED PHYSICS, vol. 66, no. 5, 1 septmbre 1989, pages 2200-2205, American Institute of Physics, New York, US; L. CHING SO et al.: "A new integrable optical modulator-switch optimized for speed and power consumption" * En entier * | 1,2,4, 11 | |
| X | GB-A-2 174 212 (STC plc) * Page 1, ligne 23 - page 4, ligne 28; figures 1-5 * | 1,2,4, 11 | |

| DOMAINES TECHNIQUES RECHERCHES (Int. Cl.5) |
|---|
| G 02 B  6/00 G 02 F  1/00 |

Le présent rapport a été établi pour toutes les revendications

| Lieu de la recherche | Date d'achèvement de la recherche | Examinateur |
|---|---|---|
| LA HAYE | 03-06-1991 | SARNEEL A.P.T. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P0402)